# EUROPEAN PATENT SPECIFICATION

(11) **EP 4 281 612 B1**
(45) Date of publication and mention of the grant of the patent: **18.02.2026**
(21) Application number: 21763133.2
(22) Date of filing: 05.08.2021
(51) Int. Cl.: D06M 11/45, A61K 9/70, D06M 11/46, D06M 11/48, D06M 11/49, D06M 13/224, D06M 23/16, D06N 3/00, D06N 3/16, D06M 11/79

(54) **TEXTILE COMPRISING CERAMIC**
KERAMIK ENTHALTENDES TEXTIL
TEXTILE COMPRENANT DE LA CÉRAMIQUE

(30) Priority: 25.01.2021 IT 202100001277
(43) Date of publication of application: 29.11.2023
(73) Proprietor: Noivita S.R.L.S., 28100 Novara (IT)
(72) Inventor: UBERTI, Francesca, 28838 Stresa (Verbano-Cusio-Ossola) (IT); MOLINARI, Claudio Giuseppe, 28100 Novara (IT)
(74) Representative: Delbarba, Andrea
(86) International application number: PCT/IB2021/057211
(87) International publication number: WO 2022/157562

(56) References cited:
- WO-A1-2015/171467
- US-A1- 2015 224 230

## Description

### FIELD OF THE INVENTION

The present invention relates to a fabric associated with at least one ceramic material comprising a mixture of castor oil and linseed oil. Furthermore, the invention relates to a patch and a textile product comprising said fabric.

### PRIOR ART

Reaching and maintaining physical wellbeing are objectives of fundamental importance for man and with modern life, characterised by numerous commitments and little time to dedicate to our body, the physical sphere tends to be affected. In this regard, reducing painful and/or inflammatory symptoms plays an important role. Inflammatory diseases include various pathologies, which may also be very different from one another, but they have in common a chronic inflammatory state indicating an anomalous immune reaction that attacks healthy cells instead of pathogenic agents. This is a constantly growing phenomenon. Thus, there is an increasing demand for interventions that may reduce the inflammatory state and therefore also reduce the painful symptoms correlated therewith. The majority of drugs used to reduce inflammation and/or pain are orally administered. The active ingredients contained in the drugs are thus absorbed and in part they pass into the bloodstream, sometimes already at the moment of entering the stomach, but substantially in the small intestine. The oral route has the advantage of being non-invasive, simple and practical for the patient. However, oral administration also has some contraindications; for example, once in the blood, the drug is conveyed by the portal vein towards the liver, where it undergoes more or less substantial transformations, which in general, have the effect of partially inactivating it, with the production of free radicals that tend to accumulate, creating the basis for triggering an inflammatory process; the part of the active ingredient that has remained intact thus reaches the general circulation. Furthermore, taking antiinflammatories by mouth causes damage, in the long run, to the gastric mucosa, which can lead to major consequences, such as the occurrence of gastritis, ulcers and perforations. There is thus a need to develop systems for administering drugs as alternatives to oral administration, but which nonetheless ensure an optimal absorption of the active ingredients. In fact, there exist numerous preparations for topical or local use for the administration of drugs, but they often do not allow a sufficient absorption of the active ingredient, which tends to be used sometimes in excessive amounts.

WO2015/171467 discloses a fabric with at least one ceramic which has been applied by a silk screen process.

Furthermore, there is an increasingly felt need to develop new approaches for reducing inflammation and/or painful symptoms also without the application of drugs.

### SUMMARY OF THE INVENTION

A first aspect of the present invention regards a fabric associated with a ceramic material, said ceramic material comprising a mixture of castor oil and linseed oil.

A second aspect of the present invention regards a patch comprising the fabric described above.

A third aspect of the present invention relates to a textile product comprising the fabric or the patch described above.

A fourth aspect of the present invention relates to a process for preparing the fabric described above.

A fifth aspect of the present invention relates to a method for reducing or treating an inflammatory pathology or for reducing painful symptoms. The method comprises at least one step of applying the fabric or the patch described above on an individual's skin.

A sixth aspect of the present invention relates to a method for local administration of an active ingredient, preferably for cutaneous administration of an active ingredient. The method comprises at least one step of applying the fabric or the patch described above on an individual's skin.

References to methods of treatment by therapy of this description are to be interpreted as references to the compound or product itself as defined in the present application, i.e textile product, fabric or patch for use in those methods, i.e. for use as an anti-inflammatory or pain-relieving product.

### BRIEF DESCRIPTION OF THE FIGURES

The present invention will be described below in detail and exemplified for purposes of non-limiting demonstration, also with the aid of the following Figures.
Figure 1 shows the plasma concentration of vitamin D3 administered in animals with a patch according to the present invention. The graph refers to the mean +/- standard deviation of five different tests;
Figure 2 shows the prediction of in vitro plasma absorption of vitamin D3, 1 hour after administration (A) and 3 hours after administration (B);
Figure 3 shows the blood glucose levels of individuals to whom a glucose solution was administered orally and of individuals to whom the same concentration of glucose was administered by means of the patch of the present invention. (* p<0.05 vs Baseline; ** p<0.05 vs 2h; ϕ P<0.05 vs 2h (glucose));
Figure 4 shows the fabric of the present invention associated with the ceramic material which is applied with a pattern that mimics blood microcirculation in the skin;
Figure 5 shows an evaluation of painful symptoms 3 and 24 hours after administration of active ingredients with a commercial patch or following the application of the patch of the present invention with ceramic material which is applied with a pattern that mimics blood microcirculation in the skin, without active ingredients (* p<0.05 vs Baseline; ** p<0.05 vs 3h; ϕ P<0.05 vs 3h ceramic);
Figure 6 shows an evaluation of the degree of absorption of curcumin in plasma via the patch of the present invention, (* p<0.05 vs Baseline; ** p<0.05 vs 12h);

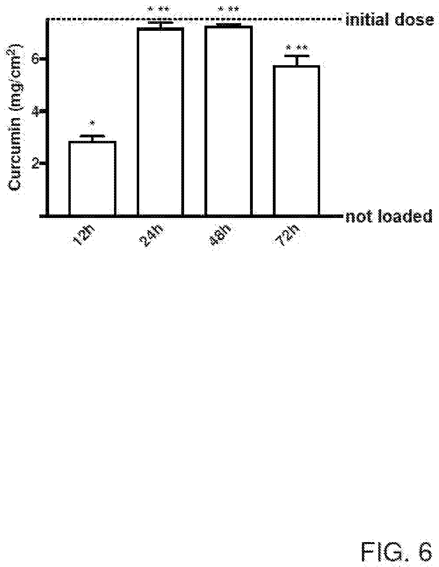

Figure 6 shows items of clothing made with the fabric of the present invention; and

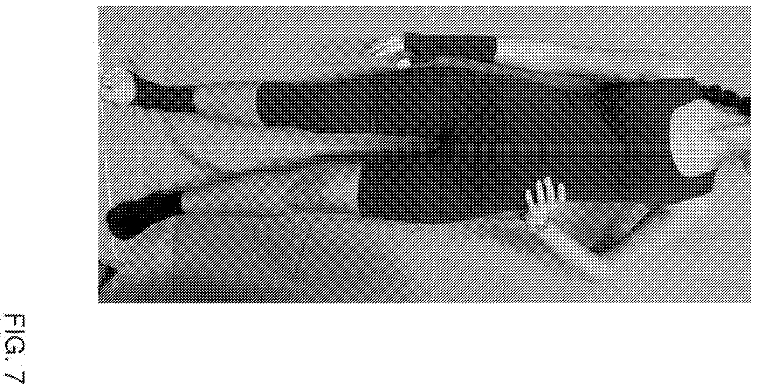

Figure 7 shows (A) the aesthetic effect and (B) the postural realignment effect obtained with an item of clothing made with the fabric of the present invention.

### DEFINITIONS

In the context of the present invention, the term "patch" means a small device, preferably adhesive, to be applied on an individual's skin.

In the context of the present invention, the term "bioavailability" means the fraction of a drug that reaches the systemic circulation without undergoing modifications, relative to the total amount of the active ingredient administered.

### DETAILED DESCRIPTION OF THE INVENTION

A first aspect of the present invention regards a fabric associated with a ceramic material, said ceramic material preferably comprising a mixture of castor oil and linseed oil.

In one embodiment, the fabric is selected among cotton, linen and a fabric comprising microfibres and/or elastane, for example a fabric comprising polyamide and/or elastane.

Preferably, the ceramic material is bonded to the fabric by printing, that is, the mixture of castor oil and linseed oil is combined with the ceramic material, which is then printed on the fabric. In other words, the ceramic material comprising the mixture of castor oil and linseed oil is printed on the fabric.

In one embodiment, the mixture of oils comprises castor oil and linseed oil, with a volumetric ratio between castor oil and linseed oil comprised between 30:70 and 5:95, preferably comprised between 40:60 and 10:90; more preferably, the volumetric ratio is 25:75, or it is 35:65.

In one embodiment, the ceramic material comprises at least two oxides selected among: Al₂O₃, Fe₂O₃, TiO₂, Cr₂O₃, SiO₂, MgO, ZrO₂, MnO₂, Co₂O₃, Y₂O₃. Preferably, the ceramic material comprises MnO₂ and Co₂O₃. In one embodiment, the ceramic material comprises MnO₂, Co₂O₃ and SiO₂.

In a preferred embodiment of the invention, the ceramic material comprises the following oxides: Al₂O₃, Fe₂O₃, TiO₂, Cr₂O₃, SiO₂, MgO, ZrO₂, MnO₂, Co₂O₃, Y₂O₃.

In one embodiment, the ceramic material comprises at least 10% weight/weight (w/w) of MnO₂ and at least 1% w/w of Co₂O₃. Furthermore, the ceramic material comprises preferably at least 5% w/w of SiO₂, preferably between 5 and the 40% w/w of SiO₂.

In one embodiment, the ceramic material is applied on the fabric with a density comprised between: 200 and 60 g/m² of fabric, preferably comprised between 190 and 70 g/m², even more preferably comprised between 180 and 60 g/m².

In one embodiment of the invention, the ceramic material is in liquid form or it is a paste.

In one embodiment of the invention, the ceramic material is mixed or loaded with at least one active ingredient. Said active ingredient is preferably a liposoluble substance, i.e. a substance that shows a reduced absorption and bioavailability when orally administered; more preferably it is selected among: vitamin D, preferably vitamin D3, curcumin, resveratrol, myrrh, serenoa repens, arnica, boswellia, lactoferrins, mineral salts and ions, preferably selected among ions of calcium, magnesium, potassium, iron and combinations thereof.

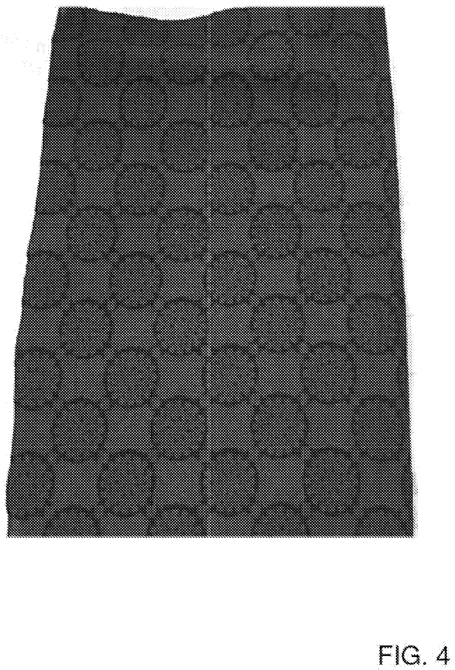

In one embodiment, the ceramic material is associated with, i.e. printed on, the fabric with a pattern that mimics skin blood microcirculation. In fact, as shown in Figure 4, the ceramic material is printed with flat shapes resembling the structure of skin microcirculation, thus depicting a plurality of cross sections of skin capillaries.

After numerous experiments, the Applicant has found that the fabric of the present invention, thanks to the combination of the ceramic material with the mixture of oils, has a pain-relieving and/or anti-inflammatory effect on the skin. Said anti-inflammatory effect is obtained even without the addition of active ingredients to the ceramic material. As shown in the example, the application of the fabric of the present invention on an individual's skin is capable of reducing painful symptoms already 3 hours after application and the greatest pain-relieving effectiveness of the fabric is observed after 24 hours. The fabric comprising the ceramic material applied with a pattern that mimics skin microcirculation, without the addition of active ingredients, has demonstrated to be particularly effective in reducing painful symptoms.

In one embodiment, the fabric of the present invention is used for the local administration of molecules with low bioavailability. In fact, thanks to the mixing of active ingredients with the ceramic material comprising the mixture of oils, it is possible to obtain an increase in the bioavailability of active ingredients that normally cannot be administered locally due to their low liposolubility. As shown in the example, the administration of active ingredients with the fabric of the present invention is wholly comparable to an oral administration of the same active ingredient. In fact, the absorption times, expressed as bioavailability, are very similar to those of normal oral administration of the active ingredient.

The Applicant has further found that the fabric of the present invention can be used for the preparation of a patch or adhesive strip for application on an individual's skin.

Therefore, a second aspect of the present invention regards a patch comprising the fabric described above in detail. In one embodiment, the patch comprises a fixing means, preferably at least one adhesive to enable the patch to be applied on an individual's skin.

The fabric of the present invention can further be used for the preparation of a textile product, such as, for example, clothing or clothing accessories. In fact, the use of the fabric described above in a textile article allows the fabric to exert an anti-inflammatory and/or pain-relieving effect on an individual who wears or uses the textile product.

A third aspect of the present invention relates to a textile product comprising the fabric or the patch described above in detail. Said textile product is preferably selected among: clothing products, preferably sportswear products, more preferably t-shirts and shorts, clothing accessories, preferably gloves, wrist sleeves and ankle sleeves, medical clothing, preferably postural shirts, corsets, knee supports, ankle supports and wrist supports.

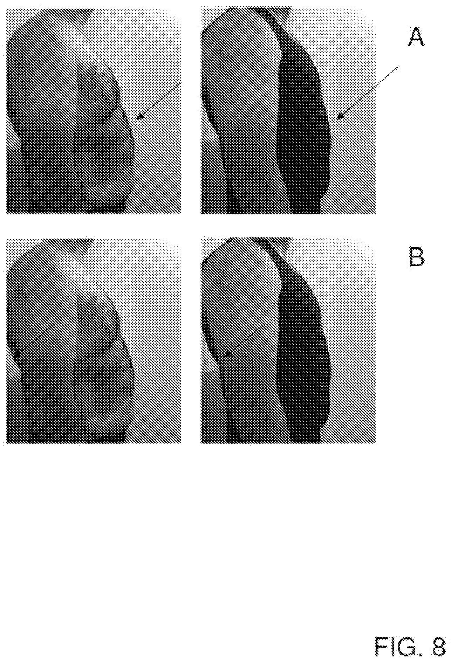

In addition to a medical action, some clothing items also have aesthetics as an added value, as they are body shaping (Figure 8).

A fourth aspect of the present invention relates to a process for preparing a fabric.

The process comprises the steps of:
a) Making a fabric available;
b) Mixing a ceramic material with a mixture of oils comprising castor oil and linseed oil; and
c) applying the ceramic material prepared in step b) on the fabric.

In one embodiment, the ceramic material obtained in step b) is applied on the fabric, preferably uniformly. In one embodiment, the ceramic material is printed on the fabric, preferably using perforated cylinders that allow a constant outflow of the ceramic material.

The fabric obtained in step c) undergoes drying, preferably at a temperature comprised between 70 and 200°C, preferably between 75 and 190°C, for a time comprised between 40 and 100 minutes, more preferably between 50 and 90 minutes.

A fifth aspect of the present invention relates to a method for reducing or treating an inflammatory pathology or for reducing or treating painful symptoms. The method comprises at least a step of applying the fabric or the patch described above in detail on an individual's skin. In a preferred embodiment, the method comprises applying on an individual's skin the fabric or the patch comprising the ceramic material as described above, without the addition of active ingredients to the ceramic material. The fabric or the patch that is applied on the individual's skin comprises the ceramic material which is associated with, i.e. printed on, the fabric with a pattern that mimics skin blood microcirculation as described above.

A sixth aspect of the present invention relates to a method for local administration of at least one active ingredient, preferably for cutaneous administration of at least one active ingredient. The method comprises at least a step of applying the fabric or the patch described above in detail on an individual's skin.

### EXAMPLE

### Evaluation of the absorption of active ingredients administered with the patch

The tests were conducted by formulating a patch with vitamin D3 (active form) formulated in a nanoemulsion (using a cold nanoemulsion with linseed oil and castor oil optimised for the active ingredient, added in a 75:25 ratio), in order to evaluate the degree of release of the ceramic material in a moist environment, where the conditions of body sweating/perspiration were mimicked.

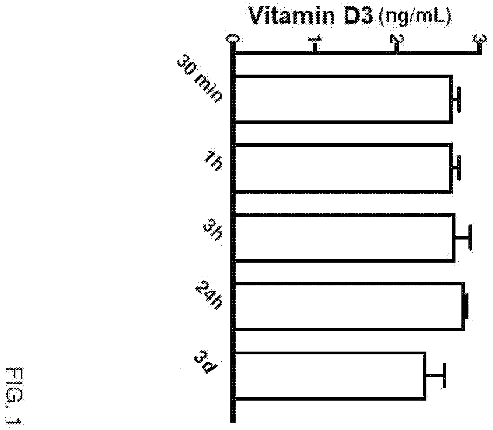

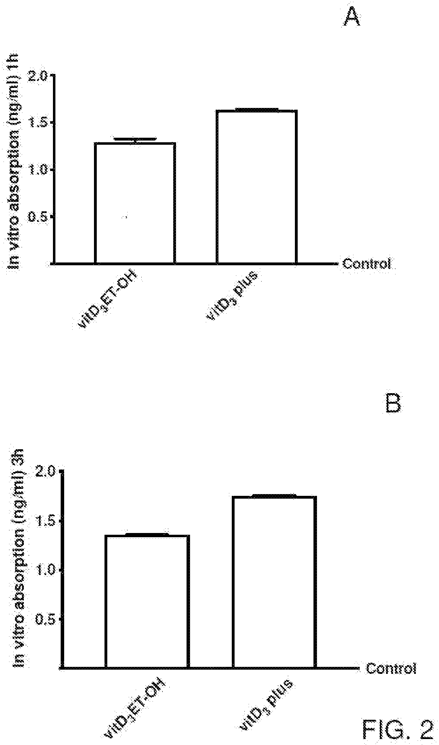

The amounts of vitamin D3 (0.003g/100g of ceramic material paste) present in the environment were measured by means of a specific kit and expressed as ng/ml. The release measurements were performed over time, up to a maximum of 3 days (Figure 1). For this test use was made of wild type C57BL/6JOIaHsd male mice (n=18, of which n=3 per time point), on which the fabric loaded with vitamin D3 was applied. The loaded fabric was maintained for 30 minutes, 1 hour, 3 hours, 24 hours and 3 days. At every time point the animal was sacrificed and bled. The plasma obtained was analysed for the quantification of vitamin D3 by means of an ELISA kit (Fn-test). The data obtained were statistically analysed and normalised based on the values obtained from control (untreated) animals (Figure 1). The data obtained were compared to those obtained in vitro (Figure 2) by mimicking the plasma concentration through an intestinal barrier model using Caco-2 in transwell plates according to the classic method (Molinari C, Morsanuto V, Ruga S, Notte F, Farghali M, Galla R, Uberti F. The Role of BDNF on Aging-Modulation Markers. Brain Sci. 2020 9;10(5):285. doi: 10.3390/brainsci10050285) for the evaluation of permeability at 1 hour (Figure 2A) and at 3 hours (Figure 2B).

The vitamin D3 assays were performed again using a kit in the basolateral compartment at the specified times and the values obtained were normalised to the control value. Two different types of vitamin D were tested, one prepared in ethanol and another for commercial use.

### Evaluation of the degree of glucose absorption

Ten healthy individuals of both sexes, aged between 25 and 35 years, were enrolled in the study.

The blood glucose measurements were performed with standard techniques, after fasting, i.e. before starting the study, and 2 hours and 4 hours after the administration of glucose, orally or by means of the patch of the present invention. The amount of glucose administered orally was the same as that administered with the patch, in order to mimic the glucose loading curve.

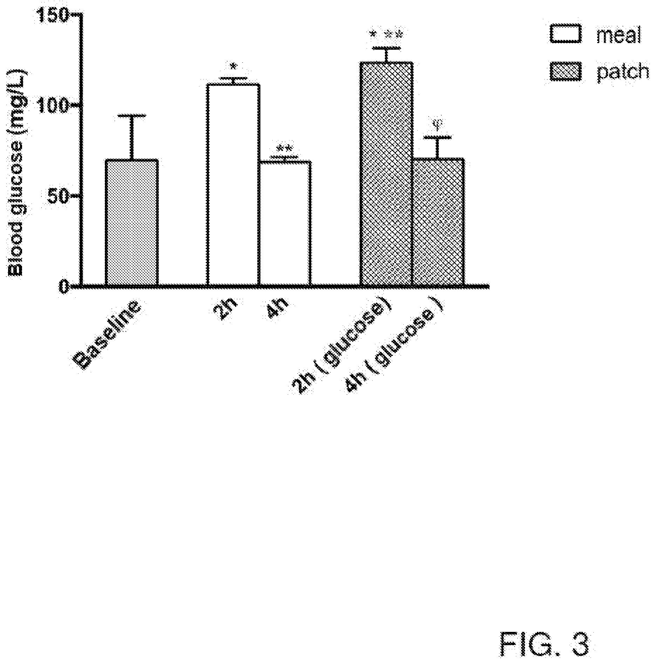

Figure 3 shows the results obtained. Administration with the patch containing glucose enables the glucose to be absorbed through the skin and to enter directly into the bloodstream.

The absorption times are similar to normal metabolization via the oral route and are in line with the parameters of a load curve for 75g of glucose (at 2h below 140mg/L).

### Evaluation of the degree of pain modulation at a local level by means of a patch

Fifteen individuals of both sexes, aged between 45 and 55 years, were enrolled.

The patch containing the ceramic material applied with a pattern that mimics skin blood microcirculation is placed directly in contact on the area with painful symptoms.

Similarly, a commercial patch comprising an active ingredient with analgesic action is placed on the area with painful symptoms.

The test involves a qualitative evaluation of the inflammatory state using a multidimensional scale called the Short-Form McGill Pain Questionnaire (SF-MGPQ), commonly used to standardise pain in adults in all chronic conditions, including rheumatic conditions. It is made up of 15 items and the pain rating index is made up of 15 representative words, evaluated on a 4-point scale: 0 "none", 1 "mild", 2 "moderate" and 3 "severe". The first 11 regard the sensorial aspect of pain, whereas items 12 to 15 regard the social aspect of pain.

The evaluation of painful symptoms using the patch of the present invention demonstrates an improvement in the situation already after 3 hours, with a greater effect observable 24 hours after application of the patch.

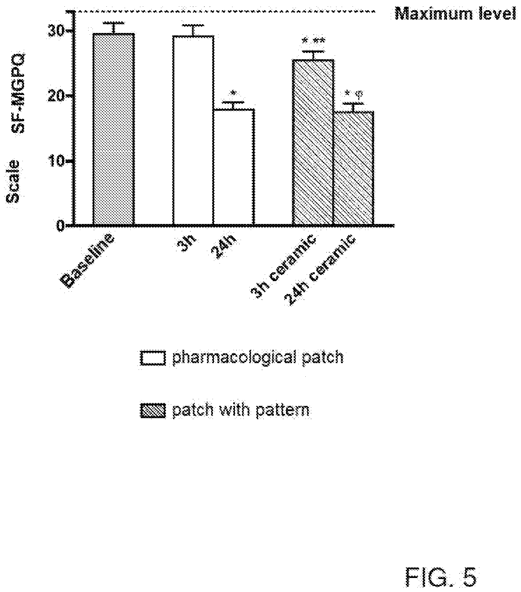

Compared to the commercial patch, the treatment with the patch of the present invention shows improvements in the painful symptoms already 3 hours after application of the patch; the maximum effect is obtained 24 hours after application of the patch (Figure 5).

### Evaluation of the degree of absorption of curcumin in plasma via the patch

The patch comprising the ceramic material according to the present invention was loaded with a dose of curcumin equal to 7.5 mg per cm² of patch.

The degree of absorption of curcumin in plasma 12, 24, 48 and 72 hours after application of the patch was evaluated. The results, shown in Figure 6, show that the absorption in plasma reaches a peak at 48 hours and, after 72 hours, the concentration of curcumin absorbed slowly begins to decrease; this data can indicate that 50% effectiveness is maintained for about 3 days.

## Claims

1. A fabric associated with at least one ceramic material comprising a mixture of castor oil and linseed oil.

2. The fabric according to claim 1, wherein the volumetric ratio between castor oil and linseed oil is comprised between 30:70 and 5:95.

3. The fabric according to claim 1 or 2, wherein the volumetric ratio between castor oil and linseed oil is comprised between 40:60 and 10:90.

4. The fabric according to any one of claims 1-3, wherein the volumetric ratio between castor oil and linseed oil is 25:75 or it is 35:65.

5. The fabric according to any one of claims 1-4, wherein the ceramic material comprises at least two oxides selected from: Al₂O₃, Fe₂O₃, TiO₂, Cr₂O₃, SiO₂, MgO, ZrO₂, MnO₂, Co₂O₃, Y₂O₃.

6. The fabric according to claim 5, wherein the ceramic material comprises MnO₂ and Co₂O₃.

7. The fabric according to claim 6, wherein the ceramic material comprises at least 10% weight/weight (w/w) of MnO₂ and at least 1% w/w of Co₂O₃.

8. The fabric according to any one of claims 1-7, wherein the ceramic material comprises at least 5% w/w of SiO₂.

9. The fabric according to claim 8, wherein the ceramic material comprises between 5 and 40% w/w of SiO₂.

10. The fabric according to any one of claims 1-9, wherein the ceramic material is applied on the fabric with a density comprised between: 200 and 60 g/m² of fabric.

11. The fabric according to claim 10, wherein the ceramic material is applied on the fabric with a density comprised between 190 and 70 g/m².

12. The fabric according to claim 11, wherein the ceramic material is applied on the fabric with a density comprised between 180 and 60 g/m².

13. The fabric according to any one of claims 1-12, wherein the ceramic material is mixed with at least one active ingredient selected from curcumin, resveratrol, myrrh, serenoa repens, arnica, boswellia, lactoferrins, mineral salts and ions preferably selected from: calcium, magnesium, potassium, iron and combinations thereof.

14. A patch comprising the fabric according to any one of claims 1-13.

15. A textile product comprising the fabric according to any one of claims 1-13 or the patch according to claim 14; said textile product is preferably selected from: clothing products, preferably sportswear products, more preferably t-shirts and shorts, clothing accessories, preferably gloves, wrist sleeves and ankle sleeves, medical clothing, preferably postural shirts, corsets, knee supports, ankle supports and wrist supports.

## Patentansprüche

1. Gewebe, das mit mindestens einem keramischen Material assoziiert ist, das eine Mischung aus Rizinusöl und Leinöl enthält.

2. Gewebe nach Anspruch 1, wobei das Volumenverhältnis zwischen Rizinusöl und Leinöl zwischen 30:70 und 5:95 liegt.

3. Gewebe nach Anspruch 1 oder 2, wobei das Volumenverhältnis zwischen Rizinusöl und Leinöl zwischen 40:60 und 10:90 liegt.

4. Gewebe nach einem der Ansprüche 1-3, wobei das Volumenverhältnis zwischen Rizinusöl und Leinöl 25:75 beträgt oder es 35:65 beträgt.

5. Gewebe nach einem der Ansprüche 1-4, wobei das keramische Material mindestens zwei Oxide enthält, die ausgewählt sind aus: Al₂O₃, Fe₂O₃, TiO₂, Cr₂O₃, SiO₂, MgO, ZrO₂, MnO₂, Co₂O₃, Y₂O₃.

6. Gewebe nach Anspruch 5, wobei das keramische Material MnO₂ und Co₂Q₃ enthält.

7. Gewebe nach Anspruch 6, wobei das keramische Material mindestens 10 Gewichtsprozent (Gew.- %) MnO₂ und mindestens 1 Gew.- % Co₂O₃ enthält.

8. Gewebe nach einem der Ansprüche 1-7, wobei das keramische Material mindestens 5 Gew.- % SiO₂ enthält.

9. Gewebe nach Anspruch 8, wobei das keramische Material zwischen 5 und 40 Gew.- % SiO₂ enthält.

10. Gewebe nach einem der Ansprüche 1-9, wobei das keramische Material auf das Gewebe mit einer Dichte aufgebracht ist, die liegt zwischen: 200 und 60 g/m² des Gewebes.

11. Gewebe nach Anspruch 10, wobei das keramische Material mit einer Dichte zwischen 190 und 70 g/m² auf das Gewebe aufgebracht ist.

12. Gewebe nach Anspruch 11, wobei das keramische Material mit einer Dichte zwischen 180 und 60 g/m² auf das Gewebe aufgebracht ist.

13. Gewebe nach einem der Ansprüche 1-12, wobei das keramische Material mit mindestens einem Wirkstoff gemischt ist, der aus Curcumin, Resveratrol, Myrrhe, Serenoa repens, Arnika, Boswellia, Lactoferrinen, Mineralsalzen und Ionen ausgewählt ist, die vorzugsweise aus Calcium, Magnesium, Kalium, Eisen und Kombinationen davon ausgewählt sind.

14. Pflaster, das das Gewebe nach einem der Ansprüche 1-13 enthält.

15. Textilprodukt, enthaltend das Gewebe nach einem der Ansprüche 1-13 oder das Pflaster nach Anspruch 14; wobei das Textilprodukt vorzugsweise ausgewählt ist aus:
Bekleidungsprodukten, vorzugsweise Sportbekleidungsprodukten, bevorzugter T-Shirts und Shorts, Bekleidungsaccessoires, vorzugsweise Handschuhen, Handgelenk- und Knöchelärmeln, medizinischer Kleidung, vorzugsweise Haltungshemden, Korsetts, Kniestützen, Knöchelstützen und Handgelenkstützen.

## Revendications

1. Tissu associé à au moins un matériau céramique comprenant un mélange d'huile de ricin et d'huile de lin.

2. Tissu selon la revendication 1, dans lequel le rapport volumétrique entre l'huile de ricin et l'huile de lin est compris entre 30:70 et 5:95.

3. Tissu selon la revendication 1 ou 2, dans lequel le rapport volumétrique entre l'huile de ricin et l'huile de lin est compris entre 40:60 et 10:90.

4. Tissu selon l'une quelconque des revendications 1-3, dans lequel le rapport volumétrique entre l'huile de ricin et l'huile de lin est de 25:75 ou de 35:65.

5. Tissu selon l'une quelconque des revendications 1-4, dans lequel le matériau céramique comprend au moins deux oxydes choisis parmi : Al₂O₃, Fe₂O₃, TiO₂, Cr₂O₃, SiO₂, MgO, ZrO₂, MnO₂, Co₂O₃, Y₂O₃.

6. Tissu selon la revendication 5, dans lequel le matériau céramique comprend du MnO₂ et du Co₂O₃.

7. Tissu selon la revendication 6, dans lequel le matériau céramique comprend au moins 10 % en poids/poids (p/p) de MnO₂ et au moins 1 % en poids/poids de Co₂O₃.

8. Tissu selon l'une quelconque des revendications 1-7, dans lequel le matériau céramique comprend au moins 5 % p/p de SiO₂.

9. Tissu selon la revendication 8, dans lequel le matériau céramique comprend entre 5 et 40 % p/p de SiO₂.

10. Tissu selon l'une quelconque des revendications 1-9, dans lequel le matériau céramique est appliqué sur le tissu avec une densité comprise entre : 200 et 60 g/m² de tissu.

11. Tissu selon la revendication 10, dans lequel le matériau céramique est appliqué sur le tissu avec une densité comprise entre 190 et 70 g/m².

12. Tissu selon la revendication 11, dans lequel le matériau céramique est appliqué sur le tissu avec une densité comprise entre 180 et 60 g/m².

13. Tissu selon l'une quelconque des revendications 1-12, dans lequel le matériau céramique est mélangé avec au moins un ingrédient actif choisi parmi la curcumine, le resvératrol, la myrrhe, le chou palmiste, l'arnica, l'essence d'oliban, les lactoferrines, les sels minéraux et les ions choisis de préférence parmi : le calcium, le magnésium, le potassium, le fer et leurs combinaisons.

14. Patch, comprenant le tissu selon l'une quelconque des revendications 1-13.

15. Produit textile, comprenant le tissu selon l'une quelconque des revendications 1-13 ou le patch selon la revendication 14 ; ledit produit textile est de préférence choisi parmi : les produits vestimentaires, de préférence les vêtements de sport, plus préférablement les t-shirts et les shorts, les accessoires vestimentaires, de préférence les gants, les bandages pour poignets et les bandages pour chevilles, les vêtements médicaux, de préférence les chemises posturales, les corsets, les genouillères, les chevillères et les bandeaux de poignets.
